# EUROPÄISCHE PATENTSCHRIFT

(11) **EP 2 845 564 B1**
(45) Veröffentlichungstag und Bekanntmachung des Hinweises auf die Patenterteilung: **09.05.2018**
(21) Anmeldenummer: 14180790.9
(22) Anmeldetag: 13.08.2014
(51) Int. Cl.: A61C 13/00, A61C 7/00

(54) **Computer-implementiertes Verfahren zum Erzeugen eines 3D-Datenmodells eines Kiefers eines Patienten**
Computer-implemented method of generating a 3D data model of the jaw of a patient
Procédé mis en oeuvre par ordinateur destiné à produire un modèle de données 3D d'une mâchoire d'un patient

(30) Priorität: 04.09.2013 DE 102013217676
(43) Veröffentlichungstag der Anmeldung: 11.03.2015
(73) Patentinhaber: CA DIGITAL GmbH, 40822 Mettmann (DE)
(72) Erfinder: Hofmann, Michael, 92318 Neumarkt in der Oberpfalz (DE); Aichinger, Florian, 80337 München (DE)
(74) Vertreter: Pellengahr, Maximilian Rudolf

(56) Entgegenhaltungen:
- US-A1- 2002 177 108
- US-A1- 2002 180 760
- US-B1- 6 514 074

## Beschreibung

Die Erfindung betrifft ein computer-implementiertes Verfahren zum Erzeugen eines 3D-Datenmodells eines Kiefers eines Patienten, wobei das 3D-Datenmodell des Kiefers wenigstens ein 3D-Teildatenmodell der Zahnkronen der sich in einer vorbestimmten Zahnstellung befindenden Zähne des Kiefers und ein 3D-Teildatenmodell des Zahnfleischs umfasst.

Bereits an dieser Stelle sei darauf hingewiesen, dass der Begriff "3D-Datenmodell" eine Vielzahl von Datenpunkten im dreidimensionalen Raum (3D-Datenpunkte) bezeichnet, welche dazu bestimmt und geeignet sind, den räumlich-körperlichen Aufbau eines Körpers zu beschreiben. Ganz allgemein steht die Abkürzung "3D" für "dreidimensional" oder auch für "im dreidimensionalen Raum".

Ferner sei darauf hingewiesen, dass der Kiefer des Patienten der Oberkiefer oder der Unterkiefer des Patienten sein kann. Die Erfindung umfasst dabei auch Verfahren, bei welchen sowohl ein 3D-Datenmodells des Oberkiefers als auch ein 3D-Datenmodells des Unterkiefers erzeugt werden. Die den Oberkiefer bzw. den Unterkiefer betreffenden Verfahrensschritte können dabei entweder nacheinander oder zeitlich ineinander verschachtelt ablaufen.

Schließlich sei auch noch darauf hingewiesen, dass in der vorliegenden Anmeldung der allgemeinsprachlich gebräuchlichere Begriff "Zahnfleisch" und der in der Fachsprache gebräuchlichere Begriff "Gingiva" gleichwertig nebeneinander verwendet werden.

Ein Verfahren der eingangs genannten Art ist beispielsweise aus der EP 1 119 308 A1 bekannt. Bei dem bekanntern Verfahren wird ausgehend von einem 3D-Datenmodell des Kiefers, welches beispielsweise durch dreidimensionales Scannen (3D-Scannen) erhalten worden ist und welches ein 3D-Teildatenmodell der Zahnkronen der sich in einer Ist-Stellung befindenden Zähne des Patienten umfasst sowie ein 3D-Teildatenmodell des tatsächlichen Zahnfleischs des Patienten, das 3D-Teildatenmodell des tatsächlichen Zahnfleischs des Patienten in Abhängigkeit von der Bewegung der Zähne auf dem von der Ist-Stellung zu einer Soll-Stellung führenden Behandlungsweg verformt. Das bekannte Verfahren hat den Nachteil sehr aufwändig zu sein.

Ferner sind aus dem gattungsbildenden Dokument US 2002/0180760 A1 und aus dem Dokument US 6,514,074 B1 Verfahren zum Berechnen von Zahnfleisch, beispielsweise basierend auf vorab gespeicherten Schablonen, sogenannten "Templates", bekannt. Ferner sei das Dokument US 2002/0177108 A1 erwähnt.

Es ist daher Aufgabe der vorliegenden Erfindung, ein Verfahren der eingangs genannten Art anzugeben, welches einfacher durchzuführen ist.

Diese Aufgabe wird erfindungsgemäß durch ein Verfahren gemäß Anspruch 1 gelöst. Erfindungsgemäß wird also an die sich in der vorbestimmten Zahnstellung befindenden Zähne des Patienten ein neues, fiktives Zahnfleisch anmodelliert, und zwar in einer von dem tatsächlichen Zahnfleisch des Patienten im Wesentlichen unabhängigen Art und Weise. Die Erfindung beruht auf der Erkenntnis, dass ein mit Zahnfleisch verkleidetes Gebiss natürlicher aussieht und dem Patienten einen realitätsnäheren Eindruck von dem Verlauf oder dem Zwischenergebnis oder dem Endergebnis der zahnorthopädischen Behandlung vermittelt als die bloße Darstellung der Zähne, dass das Hauptaugenmerk des Patienten bei der Visualisierung auf Basis des 3D-Datenmodells aber auf der Zahnstellung liegt und nicht auf dem Zahnfleisch. An der naturgetreuen bzw. realitätsnahen Darstellung des Zahnfleischs können daher leichter Abstriche gemacht werden als an der Darstellung der Zähne. Diese Erkenntnis nutzt die Erfindung aus und ersetzt die aus dem Stand der Technik bekannte aufwändige "Verformung" des 3D-Datenmodells des tatsächlichen Zahnfleischs durch das einfacher durchzuführende Anmodellieren des 3D-Datenmodells eines fiktiven Zahnfleischs an ein 3D-Datenmodell der tatsächlichen Zähne des Patienten. Einfacher ist das erfindungsgemäße Verfahren vor allem deshalb, weil nicht der gesamte Behandlungsweg von der Ist-Stellung in die vorbestimmte Stellung durchgerechnet werden muss, sondern man das 3D-Datenmodell des fiktiven Zahnfleischs ausschließlich auf Grundlage der vorbestimmten Stellung der Zähne erstellt.

Wenn vorstehend davon die Rede war, dass das fiktive Zahnfleisch von dem tatsächlichen Zahnfleisch des Patienten im Wesentlichen unabhängig ist, so ist damit gemeint, dass die dreidimensionale räumlich-körperliche Grundgestalt des fiktiven Zahnfleischs nicht von der dreidimensionalen räumlich-körperlichen Grundgestalt des tatsächlichen Zahnfleischs abgeleitet ist, sondern unabhängig von dieser in Abhängigkeit von der dreidimensionalen Anordnung der sich in der vorbestimmten Zahnstellung befindenden Zähne erzeugt wird. Gleichwohl ist es denkbar, dass nach der Erstellung des 3D-Datenmodells des fiktiven Zahnfleischs an diesem Oberflächencharakteristika des tatsächlichen Zahnfleischs vorgesehen werden. Diese Oberflächencharakteristika können beispielsweise die Oberflächenfärbung des tatsächlichen Zahnfleischs sein oder/und die Position oder/und die Größe oder/und die Gestalt von an der Oberfläche des tatsächlichen Zahnfleischs vorhandenen Strukturen, beispielsweise Wangen- und Lippenbändchen, Zahnfleischtaschen und dergleichen. Diese Strukturen dienen lediglich der Erzielung einer möglichst naturgetreuen Darstellung.

Weitere Ausgestaltungsmöglichkeiten sind Gegenstand der weiteren abhängigen Ansprüche der Erfindung.

Die Erfindung wird im Folgenden anhand der beigefügten Zeichnung näher erläutert werden. Dabei werden nicht nur die Weiterbildungsmöglichkeiten des erfindungsgemäßen Verfahrens allgemein erläutert. Vielmehr werden auch spezifische Ausgestaltungen diskutiert, wie diese Weiterbildungsmöglichkeiten in die Praxis umgesetzt werden können. Es stellt dar:
- Figur 1: eine schematische Darstellung einer Seitenansicht eines mittels eines 3D-Scans erhaltenen 3D-Datenmodells der Zähne eines Patienten in Oberkiefer und Unterkiefer jeweils mit Zahnfleisch;
- Figur 2: eine Ansicht ähnlich Figur 1, allerdings ohne Zahnfleisch;
- Figur 3: eine Ansicht ähnlich Figur 2, allerdings ergänzt um die Zahnwurzeln;
- Figur 4: eine schematische Darstellung zur Erläuterung der in den Tabellen 1 bis 15 angegebenen Parameter;
- Figur 5: eine Darstellung zur Erläuterung des zur Definition der Lage der Achsen der Wurzeläste relativ zur Zahnachse verwendeten Koordinatensystems;
- Figur 6: eine schematische Darstellung zur Erläuterung des Modellierens der Basisgingiva aus freier marginaler Gingiva und der die Zahnwurzel umgebenden Gingiva;
- Figuren 7a und 7b: zwei Draufsichten zur Erläuterung der konkaven Abflachungsabschnitte, wobei Figur 7a zwei eng nebeneinander angeordnete Zähne zeigt, während Figur 7b ein Paar Zähne mit großem Zahnabstand zeigt;
- Figur 8: eine schematische Darstellung zur Erläuterung der Gestaltung des Endes des Gingivawalls;
- Figuren 9 bis 11: drei Darstellungen zur Erläuterung des Modellierens des 3D-Datenmodells des Gaumens, wobei die Figuren 9 und 10 in einer leicht perspektivischen Ansicht von posterior gehalten sind, während Figur 11 in einer voll perspektivischen Ansicht gehalten ist; und
- Figur 12: eine Darstellung zur Erläuterung des Vorsehens von das realistische Erscheinungsbild erhöhenden, ergänzenden Strukturen am Beispiel des Gaumens.

Ausgangspunkt des erfindungsgemäßen Verfahrens kann wie beim gattungsbildenden Stand der Technik ein 3D-Scan des Oberkiefers oder/und des Unterkiefers unter Verwendung von Licht im sichtbaren Spektralbereich oder/und unter Verwendung von Strahlung in einem anderen Spektralbereich, beispielsweise Röntgenstrahlung, sein.

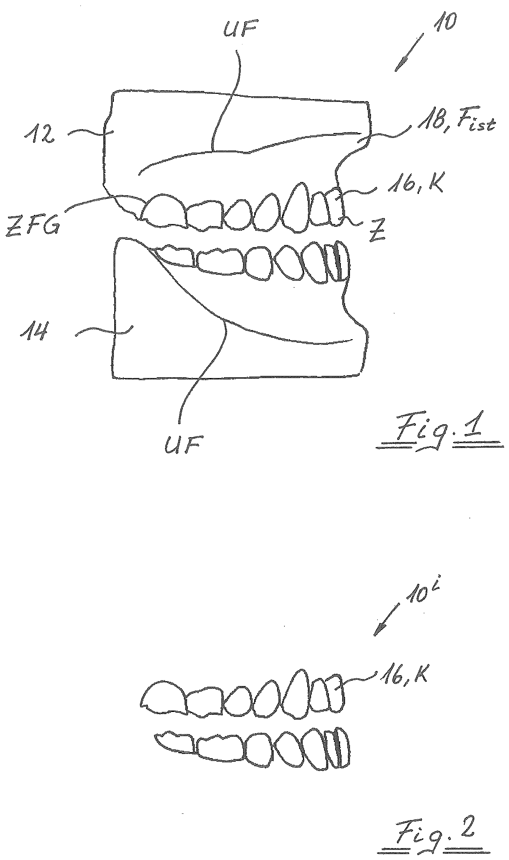

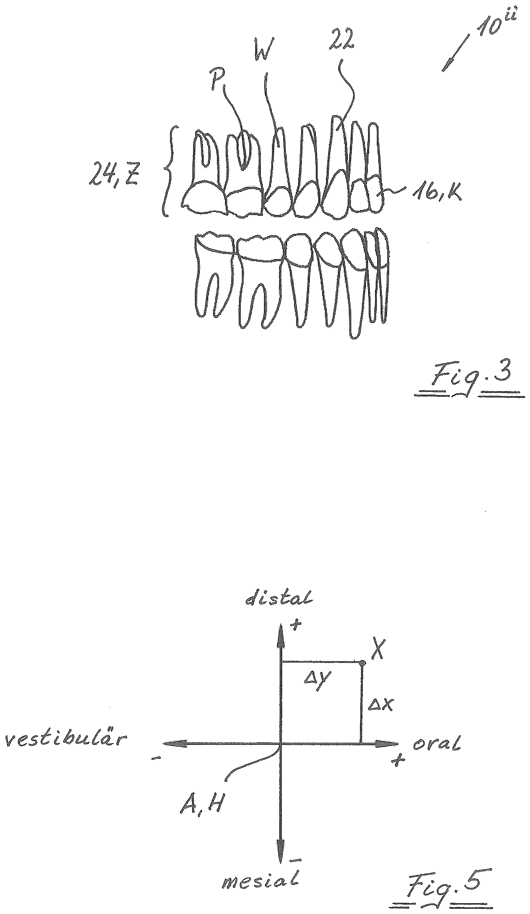

Im erstgenannten Fall, der in Figur 1 dargestellt ist, umfasst das durch den 3D-Scan erhaltene 3D-Datenmodell 10 für den Oberkiefer 12 oder/und den Unterkiefer 14 3D-Teildatenmodelle 16 für sämtliche Zahnkronen K und ein 3D-Teildatenmodell 18 des tatsächlichen Zahnfleischs Fᵢₛₜ. In diesem Fall kann zunächst für den gesamten Zahnbogen die Zahn-Zahnfleisch-Grenze ZFG ermittelt werden, um das 3D-Teildatenmodell 18 des tatsächlichen Zahnfleischs Fᵢₛₜ von den 3D-Teildatenmodellen 16 der Zahnkronen K trennen zu können. Auf diese Weise kann man ein 3D-Datenmodell 10ⁱ erhalten, das nur noch die 3D-Teildatenmodelle der Zahnkronen K umfasst (siehe Figur 2). Zu den 3D-Datenmodellen 16 der Zahnkronen K können in einem nächsten Schritt 3D-Datenmodelle 22 von Zahnwurzeln W hinzugefügt werden (siehe Figur 3). Dabei kann beispielsweise auf 3D-Teildatenmodelle eines 3D-Scans unter Verwendung von Röntgenstrahlung zurückgegriffen werden. Sollte ein derartiges 3D-Datenmodell eines Röntgenscans nicht zur Verfügung stehen, so können alternativ in einer Datenbank gespeicherte 3D-Datenmodelle von Zahnwurzeln verwendet werden, wobei als Zugriffskriterien für die Datenbank beispielsweise die Zahnposition nach dem üblichen Zahnschema der Fédération Dentaire Internationale (FDI), der Flächeninhalt der Querschnittsfläche der jeweiligen Zahnkrone K an der Zahn-Zahnfleisch-Grenze ZFG, die Zahnbreite, beispielsweise zwischen dessen Sollkontaktpunkten mit den jeweils benachbarten Zähnen, und dergleichen Parameter verwendet werden können. Schließlich ist es auch möglich, auf Grundlage der gerade als mögliche Zugriffskriterien aufgeführten Eingangsparameter 3D-Datenmodelle 22 fiktiver Wurzeln W zu erstellen.

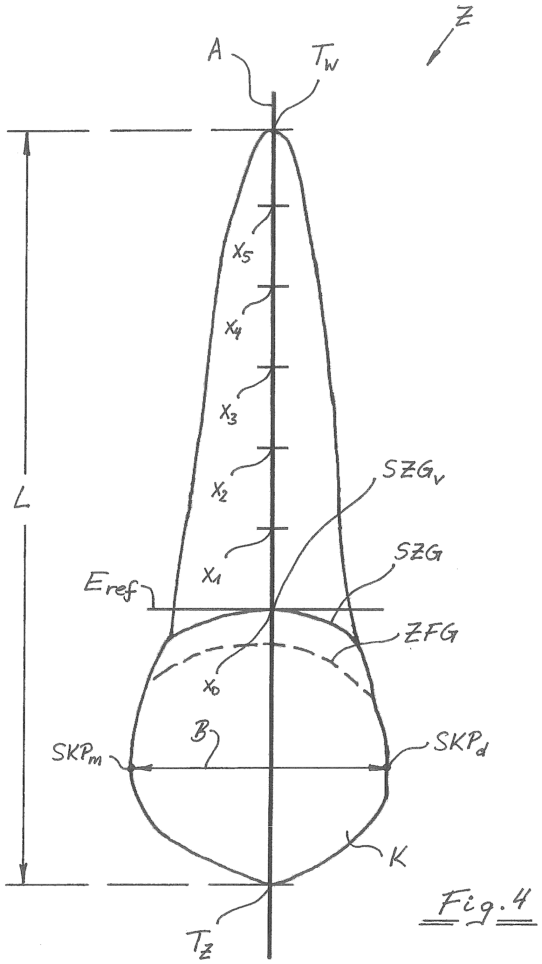

Anzumerken ist an dieser Stelle unter Bezugnahme auf Figur 4, dass die Sollkontaktpunkte SKPm, die am weitesten mesial bzw. distal gelegenen Punkte der Zahnoberfläche sind. Auf Grundlage dieser Definition kann die Zahnbreite auch für Zähne Z ermittelt werden, die keinen Nachbarzahn und somit zumindest auf einer Seite auch keinen Sollkontaktpunkt im engeren Verständnis dieses Begriffs aufweisen.

In den an das Ende der Beschreibung angefügten Tabellen 1 bis 15 sind beispielhafte Daten zur Beschreibung der typischen Gestalt der Wurzeln W der Zähne Z des zweiten Quadranten (Oberkiefer links) und des dritten Quadranten (Unterkiefer rechts) und deren jeweilige Anordnung relativ zur Zahnkrone K zusammengefasst. Für die Zähne der ersten und vierten Quadranten wird von einer spiegelsymmetrischen Anordnung zu den entsprechenden Zähnen des zweiten bzw. des dritten Quadranten ausgegangen.

Die in den Tabellen 1 bis 15 angegebenen Parameter sollen nun mit Bezug auf die Darstellung der Figur 4 erläutert werden:
In den Tabellen 1 bis 15 ist jeweils in der ersten Zeile angegeben, für welchen Zahn Z die Werte der Parametrisierung der Wurzelgestalt angegeben sind. In der zweiten Zeile ist die Breite B des jeweiligen Zahns angegeben, d.h. der Abstand zwischen dem mesialen Sollkontaktpunkt SKPm und dem distalen Sollkontaktpunkt SKPd. Die Zahnlänge L, d.h. der Abstand zwischen der Zahnspitze Tz und der Wurzelspitze Tw, ist in der dritten Zeile abgegeben. Verfügt ein Zahn über eine Mehrzahl von Wurzelästen, so wird die Zahnlänge zwischen der Zahnspitze und der Spitze des längsten Wurzelastes bestimmt. Zahnachse A ist die Verbindungslinie, die bei einwurzeligen Zähnen zwischen der Wurzelspitze Tw und der Mitte der Schneidekante, d.h. der Zahnspitze T_{z}, liegt, bei mehrwurzeligen Zähnen verläuft sie durch die Mitte der Kaufläche.

Nach dem allgemeinen Verständnis beginnt die Zahnwurzel W an der Schmelz-Zement-Grenze SZG. Diese Grenzlinie verläuft auf der von der Zahnkrone K abgewandten Seite der Zahn-Zahnfleisch-Grenze ZFG und ist somit üblicherweise von Zahnfleisch Fᵢₛₜ überdeckt. Da die Schmelz-Zement-Grenze SZG üblicherweise nicht in einer Ebene verläuft, insbesondere nicht in einer orthogonal zur Zahnachse A verlaufenden Ebene, wird im Rahmen der vorliegenden Erfindung eine durch den am weitesten verstibulär gelegenen Punkt SZGv der Schmelz-Zement-Grenze SZG und orthogonal zur Zahnachse A verlaufende Ebene als Referenzebene E_{Ref} verwendet. Der Abstand dieser Referenzebene von der Zahnspitze Tz ist in der vierten Zeile der Tabellen abgegeben.

In weiteren Zeilen der Tabellen sind die in mesio-distaler Richtung und in vestibulo-oraler Richtung bestimmten Abmessungen b der Zahnwurzel W bzw. der Wurzeläste auf unterschiedlichen Höhen der Zahnwurzel W angegeben. Die ersten Werte sind in der vorstehend erläuterten Referenzebene E_{Ref} gemessen (b(x₀ - SZGv = 0 mm)), die weiteren in Ebenen, die zur Referenzebene E_{Ref} parallel verlaufen und von dieser einen Abstand aufweisen, der ein ganzzahliges Vielfaches von 2 mm beträgt.

In der vorletzten Zeile ist der Abstand der Wurzelspitze Tw von der Referenzebene E_{Ref} angegeben, und in der letzten Zeilen finden sich ergänzende Angaben zum Verlauf der Wurzelspitze Tw relativ zur Zahnachse A.

Für Zähne, deren Wurzeln ab einer bestimmten Höhe eine Mehrzahl von Wurzelästen aufweisen, ist zudem der Abstand der Referenzebene E_{Ref} vom Furkationspunkt P (siehe Figur 3), d.h. dem Punkt, an dem sich die Wurzel in die Mehrzahl von Wurzelästen aufteilt, angegeben, sowie die Anzahl der Wurzeläste. Zudem ist für jeden Wurzelast angegeben, welchen Abstand dessen Wurzelachse X relativ zur Zahnachse A aufweist. Der Wert Δx gibt dabei den Abstand der jeweiligen Wurzelachse X von der Zahnachse A in mesio-distaler Richtung und der Wert Δy den Abstand der jeweiligen Wurzelachse X von der Zahnachse A in vestibulo-oraler Richtung an (siehe Figur 5).

Der Parametrisierung der Wurzelgestalt für den 28er-Zahn kann aus jener des 27er-Zahnes erhalten werden, indem man sämtliche Längen um 10% reduziert.

Nachzutragen ist noch, dass in den Tabellen 1 bis 15 die Parametrisierung der Wurzelgestalt jeweils für einen vorbestimmten Normzahn angegeben ist. Will man hieraus die Parametrisierung der Wurzelgestalt für einen vorgegebenen Zahn bestimmen, für den das 3D-Datenmodell der Zahnkrone K vorliegt, so bestimmt man an diesem 3D-Zahnmodell die Zahnbreite und dividiert diese durch den Wert der Zahnbreite, der in der zum betrachteten Zahn gehörenden Tabelle angegeben ist. Hierdurch erhält man einen Skalierungsfaktor, mit dem die anderen in dieser Tabelle angegebenen Längenwerte zu mulitplizieren sind.

Auf Grundlage dieser Stützwerte kann mittels eines geeigneten interpolierenden oder/und approximierenden Verfahrens, beispielsweise des Verfahrens der impliziten Oberflächen, das 3D-Datenmodell 22 jeder der Zahnwurzeln W erhalten und mit dem 3D-Datenmodell 16 der zugehörigen Zahnkrone K zu einem 3D-Datenmodell 24 des jeweiligen Zahns Z verbunden werden. Für den Fall, dass es nicht beabsichtigt sein sollte, die Zahnstellung auch ohne das anmodellierte Zahnfleisch F_{mod} darzustellen, ist es jedoch auch möglich, den Schritt des Erstellens eines 3D-Datenmodells 22 der Zahnwurzeln W auszulassen und beim Anmodellieren des Zahnfleischs F_{mod} unmittelbar von den vorstehend angesprochenen Stützwerten für die Zahnwurzeln W auszugehen. Dabei kann man in Höhenrichtung H des Zahns Z zwischen den jeweils benachbarten, bekannten Stützwerten linear interpolieren, und die Querschnittsfläche der Zahnwurzel W auf einer vorbestimmten Höhe aus den bekannten Stützwerten oder durch Linearinterpolation erhaltenen Werten als Ellipse annähern.

Im zweitgenannten Fall umfasst das durch den 3D-Scan erhaltene 3D-Datenmodell 3D-Daten sowohl der Zahnkronen K als auch der Zahnwurzeln W. Aus dem 3D-Scan im sichtbaren Spektralbereich können jedoch Informationen über den Verlauf der Zahn-Zahnfleisch-Grenze ZFG gewonnen und an dem 3D-Datenmodell berücksichtigt werden. Alternativ ist es jedoch auch möglich, Informationen über einen typischen Verlauf der Zahn-Zahnfleisch-Grenze ZFG aus einer Datenbank zu entnehmen. Bei Röntgenscannern der neuesten Generation kann es sogar möglich sein, den Verlauf der Zahn-Zahnfleisch-Grenze ZFG aus den 3D-Röntgendaten zu entnehmen.

In beiden Fällen erhält man jedoch letztlich ein 3D-Datenmodell 10ⁱⁱ, das keinerlei Informationen über die räumlich-körperliche Gestalt des tatsächlichen Zahnfleischs Fᵢₛₜ mehr enthält. Dieses 3D-Datenmodell ist es, das im Kennzeichen des Hauptanspruchs erwähnt ist, und an das das 3D-Datenmodell 26 des fiktiven Zahnfleischs F_{mod} anmodelliert wird.

Beim Erstellen des 3D-Datenmodells 26 des fiktiven Zahnfleischs F_{mod} geht man erfindungsgemäß so vor, dass man für jeden der Zähne Z des 3D-Datenmodells 10ⁱⁱ der vorbestimmten Zahnstellung gesondert ein 3D-Datenmodell 28 einer Basisgingiva BG erstellt, und die 3D-Datenmodelle 28 der Basisgingivae BG aller Zähne Z miteinander und mit dem 3D-Datenmodell 24 der vorbestimmten Zahnstellung zu einem ergänzten 3D-Datenmodell 10ⁱⁱⁱ der vorbestimmten Zahnstellung verbindet. Auf diese Weise braucht man beim Erstellen des 3D-Datenmodells 28 der Basisgingiva BG eines bestimmten Zahns Z nicht auch noch dessen absolute räumliche Orientierung und dessen räumliche Lage relativ zu den jeweils benachbarten Zähnen Z als Randbedingungen zu berücksichtigen. Dies vereinfacht das Verfahren erheblich. Die für die Erstellung der 3D-Datenmodelle 28 der Basisgingivae BG erforderliche Segmentierung des 3D-Datenmodells 10" der Zähne Z in 3D-Teildatenmodelle der einzelnen Zähne Z stellt keinen zusätzlichen Aufwand dar, da diese Segmentierung zur Planung des Behandlungswegs im Rahmen der zahnorthopädischen Behandlung ohnehin vorgenommen werden muss. Für das Erstellen des 3D-Datenmodells 28 der Basisgingiva BG jedes Zahns Z wird vorgeschlagen, dass man zumindest von wenigstens zwei charakteristischen Abmessungen der freien marginalen Gingiva F_{FMG} jedes Zahns Z und wenigstens einer charakteristischen Abmessung der die Zahnwurzel W umgebenden Gingiva F_{W} ausgeht.

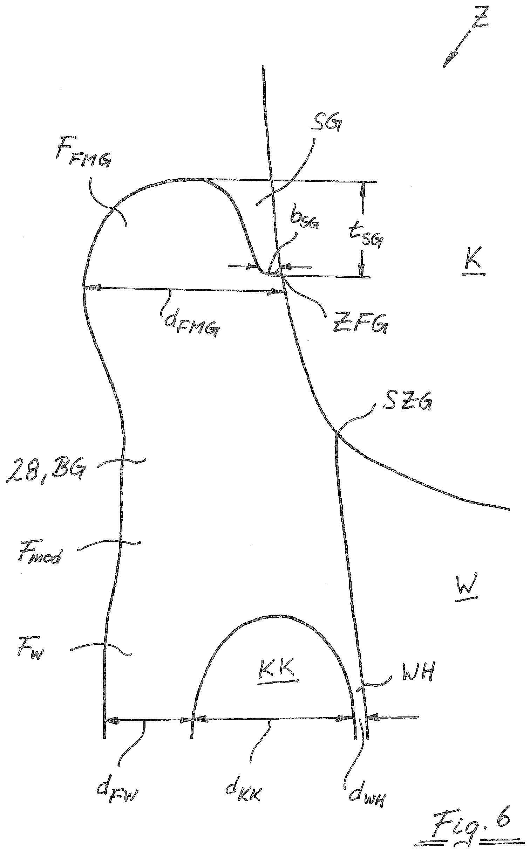

Wie in Figur 6 dargestellt ist, bildet die freie marginale Gingiva F_{FMG} einen Zahnfleischwall um den jeweiligen Zahn Z und ist von der Zahnoberfläche durch einen kleinen Graben, den so genannten Sulcus gingivalis SG, getrennt. In der Nachbarschaft des Bodens des Sulcus gingivalis SG verläuft die Zahn-Zahnfleisch-Grenze ZFG. Für die Tiefe t_{SG} des Sulcus gingivalis SG kann der Fachliteratur ein typischer Wert von 0,5 mm entnommen werden, und für die auf Höhe der Zahn-Zahnfleisch-Grenze ZFG gemessenenen Breite b_{SG} des Sulcus gingivalis SG ein Wert von etwa 0,15 mm. Beide Werte haben sich als im Zusammenhang mit dem erfindungsgemäßen Verfahren geeignete Werte erwiesen.

Da der Sulcus gingivalis SG die freie marginale Gingiva F_{FMG} auf deren zum Zahn Z hingewandten Seite begrenzt, können die Sulcus-Breite b_{SG} und die Sulcus-Tiefe t_{SG} als charakteristische Abmessungen zur Beschreibung der freien marginalen Gingiva F_{FMG} verwendet werden. Als weitere charakteristische Abmessung kann der größte Abstand d_{FMG} der Oberfläche der freien marginalen Gingiva F_{FMG} von der Zahnoberfläche verwendet werden. Dieser beträgt nach der Fachliteratur typischerweise etwa 1,6 mm. Selbstverständlich können im Zusammenhang mit der vorliegenden Erfindung auch noch weitere charakteristische Abmessungen zur Beschreibung der freien marginalen Gingiva F_{FMG} verwendet werden. Aus diesen charakteristischen Abmessungen kann man zur Durchführung des erfindungsgemäßen Verfahrens wenigstens zwei auswählen. Vorzugsweise werden sowohl die Sulcus-Breite b_{SG} als auch die Sulcus-Tiefe t_{SG} als auch der maximale Abstand d_{FMG} der Oberfläche der freien marginalen Gingiva F_{FMG} von der Zahnoberfläche verwendet.

Bei der Ermittelung der charakteristischen Abmessungen der die Zahnwurzel W umgebenden Gingiva F_{W} wird erfindungsgemäß berücksichtigt, dass die Zahnwurzel W von der Wurzelhaut WH, in der Fachsprache Desmodont genannt, umgeben ist, die nach der Fachliteratur eine typische Dicke d_{WH} von 0,1 mm aufweist. Ferner ist die vom Desmodont WH umgebene Zahnwurzel W in den Kieferknochen KK eingebettet, der nach der Fachliteratur in einem Abstand von etwa 3,5 mm von der Zahn-Zahnfleisch-Grenze ZFG eine Dicke d_{KK} von etwa 1,25 mm und an der Spitze der Zahnwurzel W eine Dicke von etwa 2,8 mm aufweist. Etwa in der Höhenmitte zwischen diesen beiden Höhenpunkten beträgt die, beispielsweise für die Zähne 13 und 23 einerseits und 33 und 43 andererseits gemittelte, Knochendicke nach der Fachliteratur etwa 0,8 mm.

Zur Dicke von Desmodont WH und Kieferknochen KK kommt noch die Dicke der Zahnfleischschicht F_{W} hinzu, die auf dem Kieferknochen KK angeordnet ist. Die Dicke d_{FW} dieser Zahnfleischschicht Fw beträgt nach der Fachliteratur vestibulär, d.h. auf der Außenseite sowohl des Oberkiefers 12 als auch des Unterkiefers 14, und lingual, d.h. auf der Innenseite des Unterkiefers 14, auf jeder Höhe ungefähr 1,0 mm. Aufgrund der Tatsache, dass das Zahnfleisch palatinal, d.h. auf der Innenseite des Oberkiefers 12, sehr schnell in den Gaumen übergeht, kann man sich hier auf das Anmodellieren der freien marginalen Gingiva F_{FMG} und das anschließende Anmodellieren des Gaumens beschränken. Letzteres erfolgt jedoch vorzugsweise erst zu einem späteren Zeitpunkt.

In Weiterbildung der Erfindung kann vorgesehen sein, dass man das 3D-Datenmodell 28 der Basisgingiva BG jedes Zahns Z unter Verwendung der durch die charakteristischen Abmessungen vorgegebenen 3D-Datenpunkte als Stützpunkte mittels eines interpolierenden und/oder aproximierenden Verfahrens erzeugt, beispielsweise mittels des Verfahrens der impliziten Oberflächen (implicit surfaces).

Das vorstehend bereits einmal angesprochene Verfahren der impliziten Oberflächen ist an sich seit Langem bekannt. Auf eines detaillierte Beschreibung dieses Verfahrens kann daher an dieser Stelle verzichtet werden. Lediglich beispielhaft sei auf den Artikel "Interpolating and Approximating Surfaces from Polygon Soup" von Chen Shen et al. in Computer Graphics Proceedings, Annual Conference Series, 2004 (ACM SIGGRAPH 2004, Los Angeles, CA, August 8-12, 2004), Seite 1 bis 9 verwiesen.

Da das 3D-Datenmodell 28 der Basisgingiva BG eines Zahns Z auf Grundlage eines 3D-Datenmodells 24 des Zahns Z einschließlich seiner Zahnwurzel W erzeugt wird, enthält es eine Vielzahl von 3D-Datenpunkten, die im Inneren des 3D-Datenmodells angeordnet und somit bei der späteren Visualisierung nicht sichtbar sind. Sofern dem Anwender des Verfahrens nicht die Möglichkeit gegeben werden soll, zwischen einer Darstellung mit Zahnfleisch und einer Darstellung ohne Zahnfleisch hin- und herzuschalten, sind diese nicht sichtbaren 3D-Datenpunkte daher im weiteren Verlauf des erfindungsgemäßen Verfahrens nicht nur entbehrlich, sondern sogar überflüssig. Zur Vereinfachung des erfindungsgemäßen Verfahrens kann daher vorgesehen sein, dass man nach dem Erstellen des 3D-Datenmodells 28 der Basisgingiva BG eines Zahns Z im Inneren dieses 3D-Datenmodells angeordnete 3D-Datenpunkte aus diesem eliminiert.

Auch infolge des Zusammenfügens der 3D-Datenmodelle 28 der Basisgingivae BG aller Zähne Z und auch des 3D-Datenmodells 10ⁱⁱ der vorbestimmten Zahnstellung zu einem gemeinsamen 3D-Datenmodell, dem ergänzten 3D-Datenmodell 10ⁱⁱⁱ, enthält das so erhaltene ergänzte 3D-Datenmodell 10ⁱⁱⁱ eine Vielzahl von 3D-Datenpunkten, die im Inneren des 3D-Datenmodells angeordnet und somit bei der späteren Visualisierung der vorbestimmten Zahnstellung für den Patienten nicht sichtbar sind. Zur Vereinfachung des erfindungsgemäßen Verfahrens wird daher vorgeschlagen, dass man beim Verbinden der 3D-Datenmodelle 28 der Basisgingivae BG aller Zähne Z miteinander und mit dem 3D-Datenmodell 10" der vorbestimmten Zahnstellung zu dem ergänzten 3D-Datenmodell 10ⁱⁱⁱ der vorbestimmten Zahnstellung im Inneren des ergänzten 3D-Datenmodells angeordnete 3D-Datenpunkte aus diesem eliminiert.

Darüber hinaus ist es möglich, dass man die 3D-Datenmodelle 28 der Basisgingiva BG einander benachbarter Zähne Z mittels 3D-Datenmodellen 30 konkaver Abflachungsabschnitte 32 verbindet. Die 3D-Datenmodelle 28 der Basisgingivae BG der Zähne Z weisen in einem zur Höhenerstreckungsrichtung H des jeweiligen Zahns Z im Wesentlichen orthogonalen Schnitt einen im Wesentlichen konvexen Verlauf der äußeren Oberfläche auf. Verbindet man die 3D-Datenmodelle 28 der Basisgingivae BG zweier benachbarter Zähne Z, so bildet sich daher dort, wo die beiden 3D-Datenmodelle 28 ineinander übergehen, eine unnatürlich aussehende keilförmige Vertiefung 34 oder es entsteht eine Lücke 36 zwischen diesen Datenmodellen 28. Diese keilförmigen Vertiefungen 34 und diese Lücken 36 werden durch die erfindungsgemäß vorgesehenen konkaven Abflachungsabschnitte 32 überdeckt, so dass sich ein naturgetreueres Erscheinungsbild ergibt.

Selbstverständlich können auch nach dem Vorsehen der konkaven Abflachungsabschnitte 32 innere 3D-Datenpunkte aus dem 3D-Datenmodell eliminiert werden.

Ferner kann vorgesehen sein, dass ein zwischen zwei benachbarten Zähnen Z vorgesehener, vestibulärer oder lingualer Abflachungsabschnitt 32 auf jeder Höhe von einer auf dieser Höhe verlaufenden und die Basisgingiva BG der beiden benachbarten Zähne Z tangential berührenden Verbindungslinie VL einen vorbestimmten Maximalabstand dₘₐₓ aufweist.

Die Einhaltung dieses vorbestimmten Maximalabstands dₘₐₓ kann ebenfalls zur Verbesserung der Naturtreue des Erscheinungsbilds des 3D-Datenmodells beitragen. Wie die Praxis zeigt, hängt der Wert des Maximalabstands dₘₐₓ, d.h. der Wert der Tiefe der konkaven Abflachungsabschnitte 32, vom Abstand der einander benachbarten Zähne Z ab. Er kann vestibulär, d.h. auf der Außenseite des Zahnbogens bei einem Zahnabstand von 0 mm, d.h. bei Zahnkontakt, etwa 1,0 mm und bei einem Zahnabstand von 7 mm etwa 1,5 mm betragen. Lingual, d.h. auf der Innenseite des Unterkiefers 14, können die konkaven Abflachungsabschnitte 32 vorzugsweise nur bis zu einem Abstand von 4 mm von der Zahn-Zahnfleisch-Grenze ZFG vorgesehen werden. Anschließend verläuft die Zahnfleischoberfläche im Wesentlichen glatt. Innerhalb des 4 mm breiten Bereichs kann der Wert des vorbestimmten Maximalabstands dₘₐₓ bei einem Zahnabstand von 0 mm, d.h. bei Zahnkontakt, etwa 0,5 mm und bei einem Zahnabstand von 7 mm etwa 1,0 mm betragen. Palatinal, d.h. auf der Innenseite des Oberkiefers 12 wird vorzugsweise nur die freie marginale Gingiva F_{FMG} berücksichtigt, nicht jedoch die Zahnwurzeln, da sich an die freie marginale Gingiva F_{FMG} der Gaumen anschließt. Daher kommt hier der vorbestimmte Maximalabstand dₘₐₓ nicht zum Einsatz. Vestibulär und lingual kann der Wert des Maximalabstands dₘₐₓ in Abhängigkeit vom Zahnabstand vorzugsweise derart ermittelt werden, dass man bei einem Zahnabstand von zwischen 0 mm und 7 mm zwischen den Maximalabstandswerten bei diesen beiden Zahnabständen linear interpoliert und dass man bei einem Zahnabstandswert von mehr als 7 mm den Maximalabstandswert für den Zahnabstand von 7 mm beibehält.

In analoger und daher nicht gesondert zeichnerisch dargestellter Weise kann auch okklusal ein konkaver Abflachungsabschnitt zwischen einander benachbarten Zähnen Z vorgesehen werden, und zwar vorzugsweise derart, dass ein zwischen zwei benachbarten Zähnen Z vorgesehener, okklusaler Abflachungsabschnitt von einer auf den oberen Rändern der freien marginalen Gingiva F_{FMG} der beiden benachbarten Zähne aufliegenden Verbindungslinie einen vorbestimmten Maximalabstand aufweist. Dieser vertikale bzw. okklusale Maximalabstand kann in Abhängigkeit vom Zahnabstand zunächst linear ansteigen, bis er bei einem Zahnabstand von 7 mm einen Wert von 0,5 mm erreicht, und anschließend bei Zahnabständen von mehr als 7 mm bei diesem Wert von 0,5 mm verharren.

Ein weiteres Merkmal, das Einfluss auf die Naturtreue der Darstellung des 3D-Datenmodells 10ⁱⁱⁱ der vorbestimmten Zahnstellung haben kann, ist der Abschluss des Gingivawalls an den beiden Enden des Zahnbogens. Daher wird in Weiterbildung der Erfindung vorgeschlagen, dass die Gingiva distal der beiden endständigen Zähne Z_{end} des jeweiligen Kiefers 12 bzw. 14 um eine vorbestimmte Länge l_{end} fortgesetzt wird, vorzugsweise in einem vorbestimmten Abstand h_{end} von der Okklusalebene OE und mit einer vorbestimmten Dicke b_{end}.

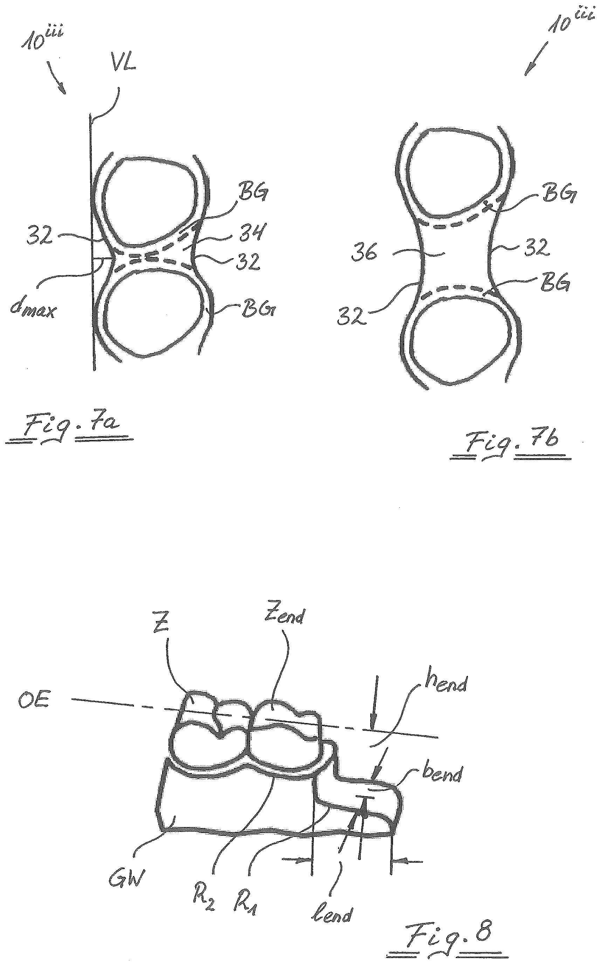

Mit bezug auf Figur 8 kann die vorbestimmte Länge l_{end} dabei vorzugsweise ausgehend von der distalsten Position der Oberfläche des distalsten Zahns Z_{end} gemessen werden. Ist einer der 7er-Zähne oder einer der 8er-Zähne der distalste Zahn Z_{end} des betrachteten Zahnbogens ZB, so kann die vorbestimmte Länge l_{end} beispielsweise 3 mm betragen. Ist in der jeweils anderen Zahnbogenhälfte der distalste Zahn Z_{end}, und in der Reihenfolge von distal nach proximal gegebenfalls weitere Zähne, nicht vorhanden, so kann die vorbestimmte Länge l_{end} für diese Zahnbogenhälfte um einen Wert erhöht werden, der gleich der in Richtung des Zahnbogens gemessenen Länge des in der anderen Zahnbogenhälfte vorhandenen entsprechenden Zahns Z, gegebenenfalls der entsprechenden Zähne, ist. Ist in beiden Zahnbogenhälften der 7er-Zahn, und in der Reihenfolge von distal nach proximal gegebenfalls weitere Zähne, nicht vorhanden, so kann die vorbestimmte Länge l_{end} für beide Zahnbogenhälften um einen Wert erhöht Werden, der gleich der in Richtung des Zahnbogens gemessenen Länge eines typischen 7er-Zahns, und gegebenenfalls der weiteren Zähne, ist. Diese typische Länge kann beispielsweise der Fachliteratur entnommen werden.

Was den vorbestimmten Abstand h_{end} anbelangt, so kann der okklusale Rand R₁ des Gingivawalls GW um beispielsweise etwa 3 mm weiter von der Okklusionsebene OE weg verlaufen als der distale Abschnitt des okklusalen Randes R₂ der freien marginalen Gingiva F_{FMG} des distalsten Zahnes Z_{end} der jeweiligen Zahnbogenhälfte. Ferner kann die vorbestimmte Dicke b_{end} gleich der um beispielsweise 1 mm verringerten Maximaldicke der Gingiva des distalsten Zahns sein, wobei die Dickenverringerung vorzugsweise gleichmäßig auf die vestibuläre und die orale Seite aufgeteilt werden kann.

Was die von der Okklusionsebene OE ferne Grenze der Erzeugung des 3D-Datenmodells 10ⁱⁱⁱ anbelangt, kann vorgesehen sein, dass man die Gingiva F_{mod} nur bis zur Umschlagfalte UF modelliert. Die Umschlagfalte UF (siehe Figur 1) ist dort angeordnet, wo das Zahnfleisch in das Lippenfleisch übergeht. Die Lage der Umschlagfalte UF kann beispielsweise dem 3D-Scan im sichtbaren Spektralbereich entnommen werden. Es ist jedoch auch möglich, die Lage der Umschlagfalte UF auf Grundlage von Daten festzulegen, welche der Fachliteratur entnommen sind. In der Praxis hängen die Werte für den Abstand der Umschlagfalte UF von der Okklusionsebene OE von der jeweiligen Zahnposition ab. So beträgt dieser Abstandswert im Oberkiefer 12 für die 1er-Zähne 25 mm, für die 2er-Zähne 24,5 mm, für die 3er-Zähne 23 mm, für die 4er-Zähne 21 mm, für die 5er-Zähne 20 mm, für die 6er-Zähne 16,5 mm, für die 7er-Zähne 15 mm und für die 8er-Zähne 12,5 mm, während er im Unterkiefer 14 für die 1er-Zähne 23 mm, für die 2er-Zähne 23 mm, für die 3er-Zähne 22 mm, für die 4er-Zähne 21,5 mm, für die 5er-Zähne 20 mm, für die 6er-Zähne 19 mm, für die 7er-Zähne 17 mm und für die 8er-Zähne 16 mm beträgt.

In Weiterbildung der Erfindung wird ferner vorgeschlagen, dass man für den Oberkiefer 12 ein 3D-Datenmodell 38 des sich zwischen den beiden Zahnbogenhälften erstreckenden Gaumens G erstellt.

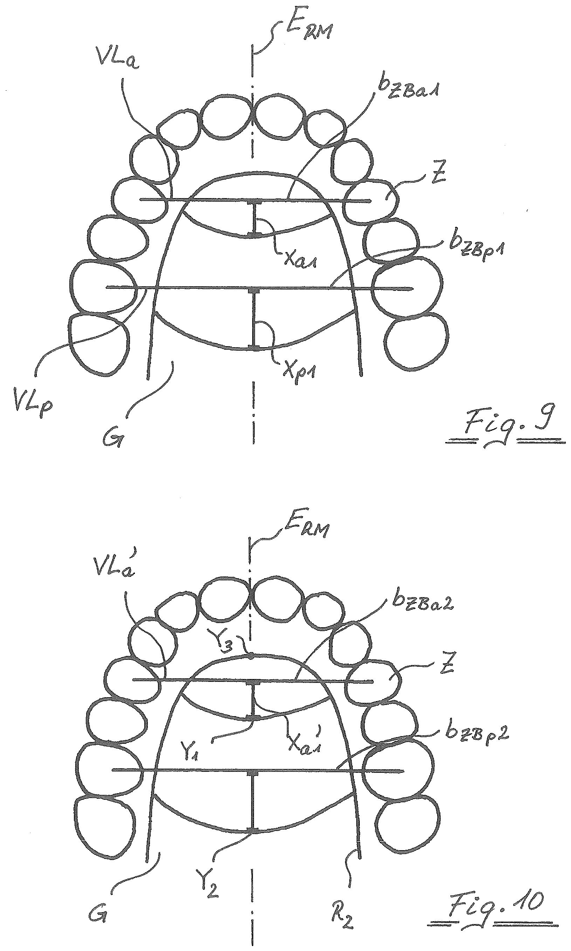

Hierzu kann man mit Bezug auf die Figuren 9 bis 11 beispielsweise wie folgt vorgehen:
In einem ersten Schritt bestimmt man am 3D-Datenmodell der Ist-Stellung der Zähne Z für die beiden 4er-Zähne des Oberkiefers 12 die sogenannte vordere oder anteriore Zahnbogenbreite b_{ZBa1} (Figur 9). Als Ausgangspunkt für diese Messung kann dabei die Mitte der Fissur der 4er-Zähne, d.h. der ersten oberen Prämolaren, verwendet werden:
In einem zweiten Schritt bestimmt man auch am 3D-Datenmodell 10ⁱⁱⁱ der jeweils betrachteten Stellung der Zähne Z des Patienten die vordere Zahnbogenbreite b_{ZBa2} (Figur 10).

Anschließend bildet man in einem dritten Schritt durch Division der vorderen Zahnbogenbreite b_{ZBa2} der jeweils betrachteten Zahnstellung durch die vordere Zahnbogenbreite b_{ZBa1} der Ist-Zahnstellung einen vorderen Zahnbogenbreitenquotienten Q_{ZBa}.

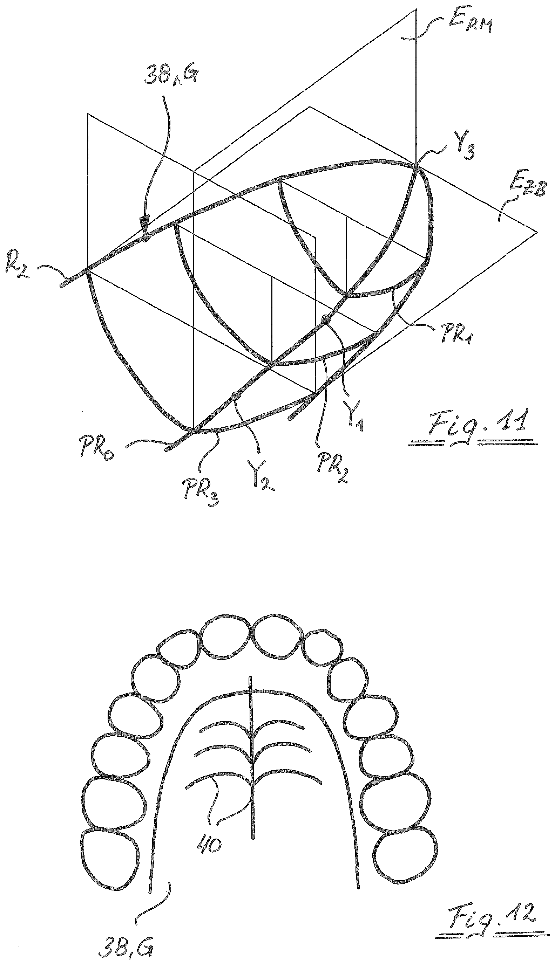

In einem vierten Schritt wird auf Höhe der Raphe-Median-Ebene E_{RM} (siehe auch Figur 11) in einer orthogonal zur Zahnbogenebene E_{ZB} (siehe Figur 11) verlaufenden Richtung der Abstand xₐ₁ der Verbindungslinie VLa der Ausgangspunkte der beiden 4er-Zähne von dem Gaumenabschnitt des 3D-Datenmodells der Ist-Stellung der Zähne bestimmt.

In einem fünften Schritt multipliziert man diesen Abstand xₐ₁ mit dem vorderen Zahnbogenbreitenquotienten Q_{ZBa}, um einen Modellabstand xₐ₁' des zu modellierenden Gaumens G von der Verbindungslinie VLa' der Ausgangspunkte der beiden 4er-Zähne der jeweils betrachteten Zahnstellung zu ermitteln.

Lässt man diesen Modellabstand xₐ₁' in einem sechsten Schritt von der Mitte der Verbindungslinie VIa' der Ausgangspunkte der beiden 4er-Zähne der jeweils betrachteten Zahnstellung ausgehen, so erhält man einen ersten Stützpunkt Y₁ für die Modellierung des zu modellierenden Gaumens G (siehe auch Figur 11).

Nun wiederholt man die ersten bis sechsten Schritte für die beiden 6er-Zähne, d.h. für die hintere oder posteriore Zahnbogenbreite b_{ZBp1} bzw. b_{ZBp2}, und erhält so einen zweiten Stützpunkt Y2 für die Modellierung des zu modellierenden Gaumens G (siehe auch Figur 11). Als Ausgangspunkt für die Messung der Zahnbogenbreite verwendet man dabei den Kreuzungspunkt der vorderen Längsfissur mit der bukkalen Querfissur der 6er-Zähne.

Einen dritten Stützpunkt Y₃ für die Modellierung des zu modellierenden Gaumens G bildet der Schnittpunkt der Raphe-Median-Ebene E_{RM} mit dem inneren Rand R₂ der bereits modellierten freien marginalen Gingiva F_{FMG}, und zwar in einer vorbestimmten Höhe oberhalb der Zahn-Zahnfleisch-Grenze ZFG, beispielsweise in einer Höhe von 1,5 mm oberhalb der Zahn-Zahnfleisch-Grenze ZFG.

Die so erhaltenen drei Stützpunkte Y₁, Y₂ und Y₃ verbindet man in einem weiteren Schritt mittels eines nach Art eines Schiffsbugs verlaufenden gekrümmten Profils PR₀.

Ausgehend von dem dritten Stützpunkt Y3 bestimmt man in einem weiteren Schritt eine Mehrzahl von zueinander parallel verlaufenden Ebenen, die sowohl zur Raphe-Median-Ebene E_{RM} als auch zur Zahnbogenebene E_{ZB} orthogonal verlaufen und voneinander vorbestimmte Abstände aufweisen, die vorzugsweise alle den gleichen Wert aufweisen. Jede dieser Ebenen schneidet zum einen das nach Art eines Schiffsbugs verlaufende Profil PR₀ und zum anderen auf beiden Hälften des Zahnbogens ZB den inneren Rand der bereits modellierten freien marginalen Gingiva F_{FMG}, und zwar in einer vorbestimmten Höhe oberhalb der Zahn-Zahnfleisch-Grenze ZFG, beispielsweise in einer Höhe von 1,5 mm oberhalb der Zahn-Zahnfleisch-Grenze ZFG. Für jede dieser Ebenen verbindet man die drei Schnittpunkte mittels einer zur Raphe-Median-Ebene symmetrischen gekrümmten Bogenlinie PR₁, PR2, ... Der übersichtlicheren Darstellung halber ist in Figur 11 lediglich die in einer vorbestimmten Höhe oberhalb der Zahn-Zahnfleisch-Grenze ZFG verlaufende Linie R₂ des Rands der bereits modellierten freien marginalen Gingiva F_{FMG} dargestellt.

Das nach Art eines Schiffsbugs verlaufende gekrümmte Profil PR₀ und die Mehrzahl der zur Raphe-Median-Ebene E_{RM} symmetrischen gekrümmten Bogenlinien PR₁, PR₂, ... bilden ein Gerüst von Datenpunkten, das als Ausgangspunkt für die Modellierung des Gaumens G mittels eines interpolierenden und/oder approximierenden Verfahrens, beispielsweise mittels des Verfahrens der impliziten Oberflächen, dient.

Zur Erhöhung der Naturtreue der Darstellung kann es im Falle der Darstellung des Oberkiefers 12 ferner vorteilhaft sein, dass man am 3D-Datenmodell 38 des Gaumens G wenigstens eine ergänzende Struktur 40 vorsieht, welche beispielsweise die Papilla incisiva oder/und die Plicae transversae oder/und die Satura palatina oder/und das Lippenbändchen oder/und wenigstens eines der Wangenbändchen repräsentiert.

In analoger Weise kann es zur Erhöhung der Naturtreue der Darstellung im Falle der Darstellung des Unterkiefers 14 ferner vorteilhaft sein, dass man am 3D-Datenmodell des Unterkiefers 14 wenigstens eine ergänzende Struktur vorsieht, welche beispielsweise das Zungenbändchen oder/und das Lippenbändchen oder/und wenigstens eines der Wangenbändchen repräsentiert.

Wie eingangs bereits erwähnt, kann es zusätzlich oder alternativ sowohl für die Darstellung des Oberkiefers 12 als auch für die Darstellung des Unterkiefers 14 vorteilhaft sein, wenn man ferner Oberflächencharakteristika des tatsächlichen Zahnfleischs Fᵢₛₜ vorsieht, vorzugsweise an der Oberfläche des tatsächlichen Zahnfleischs Fᵢₛₜ vorhandene Strukturen, beispielsweise Warzen, Zahnfleischtaschen und dergleichen.

An dieser Stelle sei nochmals darauf hingewiesen, dass nach jeder Veränderung des 3D-Datenmodells 10ⁱⁱⁱ innere 3D-Datenpunkte aus dem 3D-Datenmodell eliminiert werden können. Dieses Eliminieren von 3D-Datenpunkten kann sich dabei auf das Zahnfleisch F_{mod} repräsentierende 3D-Datenpunkte beschränken, so dass beispielsweise 3D-Datenpunkte, welche die Zahnwurzeln W repräsentieren, beibehalten werden können, um zwischen verschiedenen Darstellungsarten wechseln zu können.

Schließlich kann das mithilfe des erfindungsgemäßen Verfahrens erzeugte 3D-Datenmodell 10ⁱⁱⁱ der vorbestimmten Zahnstellung eingefärbt werden, wobei dem 3D-Teildatenmodell der Zähne Z angehörende Oberflächenabschnitte mit einer Zahnfärbung und dem 3D-Teildatenmodell des Zahnfleischs F_{mod} angehörende Oberflächenabschnitte mit einer Zahnfleischfärbung belegt werden können. Dabei können die Zahnfärbung oder/und die Zahnfleischfärbung als weitere Oberflächencharakteristika dem durch einen 3D-Scan im sichtbaren Spektralbereich erhaltenen 3D-Datenmodell 10 entnommen sein. In diesem Zusammenhang sei ferner darauf hingewiesen, dass der Begriff "Färbung" im Zusammenhang mit der vorliegenden Erfindung nicht nur eine gleichförmige Färbung mit einer einzigen Farbe umfasst, sondern auch das Belegen mit einem Wechselspiel einer Mehrzahl von Farben oder/und hellerer und dunklerer Farbtöne, beispielsweise zur Darstellung einer vorbestimmten Oberflächentextur.

**Tabelle 1:**

| | | |
|---|---|---|
| Zahn | 21 | |
| Breite (Sollkontaktpkte.) | 9,0 mm | |
| Zahnlänge | 25,0 mm | |
| d(Zahnspitze - SZGv) | 10,0 mm | |

| | mesio-distal | vestibulo-oral |
|---|---|---|
| d(x₀ - SZGv) = 0 mm | 6,0 mm | 7,0 mm |
| d(x₁ - SZGv) = 2 mm | 5,7 mm | 6,8 mm |
| d(x₂ - SZGv) = 4 mm | 5,3 mm | 6,4 mm |
| d(x₃ - SZGv) = 6 mm | 4,7 mm | 6,0mm |
| d(x₄ - SZGv) = 8 mm | 4,2 mm | 5,2 mm |
| d(x5 - SZGv) = 10 mm | 3,5 mm | 4,4 mm |
| d(x₆ - SZGv) = 12 mm | 2,3 mm | 3,4 mm |
| d(x₇ - SZGv) = 14 mm | 1,8 mm | 2,4 mm |
| d(Wurzelspitze - SZGv) | 15,0 mm | |
| Wurzelmerkmal | Die letzten 2 mm 7° nach distal geneigt | |

**Tabelle 2:**

| | | |
|---|---|---|
| Zahn | 22 | |
| Breite (Sollkontaktpkte.) | 7,1 mm | |
| Zahnlänge | 24,0 mm | |
| d(Zahnspitze - SZGv) | 9,5 mm | |

| | mesio-distal | vestibulo-oral |
|---|---|---|
| d(x₀ - SZGv) = 0 mm | 4,5 mm | 6,5 mm |
| d(x₁ - SZGv) = 2 mm | 4,4 mm | 6,3 mm |
| d(x₂ - SZGv) = 4 mm | 3,8 mm | 6,1 mm |
| d(x₃ - SZGv) = 6 mm | 3,6 mm | 6,0 mm |
| d(x₄ - SZGv) = 8 mm | 3,3 mm | 5,4 mm |
| d(x₅ - SZGv) = 10 mm | 2,8 mm | 4,9 mm |
| d(x₆ - SZGv) = 12 mm | 2,3 mm | 3,8 mm |
| d(x₇ - SZGv) = 14 mm | 1,5 mm | 2,7 mm |
| d(Wurzelspitze - SZGv) | 14,5 mm | |
| Wurzelmerkmal | Die letzten 2 mm 7° nach distal geneigt | |

**Tabelle 3:**

| | | |
|---|---|---|
| Zahn | 23 | |
| Breite (Sollkontaktpkte.) | 8,0 mm | |
| Zahnlänge | 26,5 mm | |
| d(Zahnspitze - SZGv) | 10,5 mm | |

| | mesio-distal | vestibulo-oral |
|---|---|---|
| d(x₀ - SZGv) = 0 mm | 5,3 mm | 8,0 mm |
| d(x₁ - SZGv) = 2 mm | 5,2 mm | 7,8 mm |
| d(x₂ - SZGv) = 4 mm | 4,8 mm | 7,4 mm |
| d(x₃ - SZGv) = 6 mm | 4,2 mm | 6,9 mm |
| d(x₄ - SZGv) = 8 mm | 3,8 mm | 5,7 mm |
| d(x₅ - SZGv) = 10 mm | 3,3 mm | 4,6 mm |
| d(x₆ - SZGv) = 12 mm | 2,8 mm | 3,8 mm |
| d(x₇ - SZGv) = 14 mm | 2,2 mm | 2,4 mm |
| d(Wurzelspitze - SZGv) | 16,0 mm | |
| Wurzel merkmal | Die letzten 2 mm 7° nach distal geneigt | |

**Tabelle 4:**

| | | | | |
|---|---|---|---|---|
| Zahn | 24 | | | |
| Breite (Sollkontaktpkte.) | 7,7 mm | | | |
| Zahnlänge | 22,5 mm | | | |
| d(Zahnspitze - SZGv) | 8,2 mm | | | |

| | mesio-distal | | vestibulo-oral | |
|---|---|---|---|---|
| d(x₀ - SZGv) = 0 mm | 4,6 mm | | 9,3 mm | |
| d(x₁ - SZGv) = 2 mm | 4,4 mm | | 9,0 mm | |
| d(x₂ - SZGv) = 4 mm | 3,9 mm | | 9,0 mm | |
| d(x₃ - SZGv) = 6 mm | 3,4 mm | | 8,5 mm | |
| d(SZGv - Furkation) | 7,5 mm | | | |
| Anzahl der Wurzeln | 2 | | | |

| | Wurzel 1 | | Wurzel 2 | |
|---|---|---|---|---|
| | mesio-distal | vestibulo-oral | mesio-distal | vestibulo-oral |
| Wurzelachse (Δx,Δy) | 0,0 mm | -2,0 mm | 0,0 mm | +2,0 mm |
| d(x₄ - SZGv) = 8 mm | 2,9 mm | 3,9 mm | 2,9 mm | 3,4mm |
| d(x₅ - SZGv) = 10 mm | 2,8 mm | 3,2 mm | 2,8 mm | 2,6 mm |
| d(x₆ - SZGv) = 12 mm | 2,1 mm | 2,3 mm | 2,0 mm | 1,7 mm |
| d(Wurzelspitze - SZGv) | 13,7 mm | | 14,3 mm | |
| Wurzelmerkmal | Die letzten 4 mm 7° nach oral geneigt | | --- | |

**Tabelle 5:**

| | | |
|---|---|---|
| Zahn | 25 | |
| Breite (Sollkontaktpkte.) | 6,9 mm | |
| Zahnlänge | 21,5 mm | |
| d(Zahnspitze - SZGv) | 7,0 mm | |

| | mesio-distal | vestibulo-oral |
|---|---|---|
| d(x₀ - SZGv) = 0 mm | 4,3 mm | 9,2 mm |
| d(x₁ - SZGv) = 2 mm | 4,2 mm | 8,8 mm |
| d(x₂ - SZGv) = 4 mm | 3,3 mm | 8,7 mm |
| d(x₃ - SZGv) = 6 mm | 3,0 mm | 7,5 mm |
| d(x₄ - SZGv) = 8 mm | 2,7 mm | 6,4 mm |
| d(x₅ - SZGv) = 10 mm | 2,6 mm | 5,1 mm |
| d(x₆ - SZGv) = 12 mm | 2,0 mm | 4,0 mm |
| d(x₇ - SZGv) = 14 mm | 1,3 mm | 2,0 mm |
| d(Wurzelspitze - SZGv) | 14,5mm | |
| Wurzelmerkmal | Die letzten 2 mm 15° nach distal geneigt | |

**Tabelle 6:**

| | | | | | | |
|---|---|---|---|---|---|---|
| Zahn | 26 | | | | | |
| Breite (Sollkontaktpkte.) | 8,0 mm | | | | | |
| Zahnlänge | 20,3 mm | | | | | |
| d(Zahnspitze - SZGv) | 8,9 mm | | | | | |
| | mesio-distal | | | vestibulo-oral | | |
| d(x₀ - SZGv) = 0 mm | 7,8 mm | | | 11,4 mm | | |
| d(x₁ - SZGv) = 2 mm | 7,7 mm | | | 11,4 mm | | |
| d(SZGv - Furkation) | 3,5 mm | | | | | |
| Anzahl der Wurzeln | 3 | | | | | |

| | Wurzel 1 | | Wurzel 2 | | Wurzel 3 | |
|---|---|---|---|---|---|---|
| | mesio-distal | vestibulo-oral | mesio-distal | vestibulo-oral | mesio-distal | vestibulo-oral |
| Wurzelachse (Δx,Δy) | -2,5 mm | -4,0 mm | +2,5 mm | -4,0 mm | 0,0 mm | +5,0 mm |
| d(x₂ - SZGv) = 4 mm | 3,5 mm | 7,0 mm | 3,4 mm | 5,7 mm | 5,7 mm | 4,3 mm |
| d(x3 - SZGv) = 6 mm | 3,0 mm | 6,2mm | 2,8 mm | 4,8 mm | 5,4 mm | 3,8 mm |
| d(x₄ - SZGv) = 8 mm | 2,8 mm | 5,3 mm | 2,3 mm | 4,1 mm | 4,4 mm | 3,5 mm |
| d(x₅ - SZGv) = 10 mm | 2,4 mm | 4,1 mm | 2,2 mm | 3,4 mm | 3,5 mm | 2,8 mm |
| d(Wurzelspitze - SZGv) | 11,4 mm | | 11,4 mm | | 12,0 mm | |
| Wurzelmerkmal | --- | | Die letzten 4 mm 7° nach mesial geneigt | | --- | |

**Tabelle 7:**

| | | | | | | |
|---|---|---|---|---|---|---|
| Zahn | 27 | | | | | |
| Breite (Sollkontaktpkte.) | 9,4 mm | | | | | |
| Zahnlänge | 18,0 mm | | | | | |
| d(Zahnspitze - SZGv) | 6,0 mm | | | | | |
| | mesio-distal | | | vestibulo-oral | | |
| d(x₁ - SZGv) = 2 mm | 6,9 mm | | | 9,8 mm | | |
| d(SZGv - Furkation) | 3,0 mm | | | | | |
| Anzahl der Wurzeln | 3 | | | | | |

| | Wurzel 1 | | Wurzel 2 | | Wurzel 3 | |
|---|---|---|---|---|---|---|
| | mesio-distal | vestibulo-oral | mesio-distal | vestibulo-oral | mesio-distal | vestibulo-oral |
| Wurzelachse (Δx,Δy) | -0,5 mm | -3,0 mm | +2,4 mm | -3,0 mm | 0,0 mm | +3,5 mm |
| d(x₂ - SZGv) = 4 mm | 3,4 mm | 6,0 mm | 3,2 mm | 5,0 mm | 5,2 mm | 4,3 mm |
| d(x3 - SZGv) = 6 mm | 2,5 mm | 5,3 mm | 2,8 mm | 4,4 mm | 4,5 mm | 3,8 mm |
| d(x₄ - SZGv) = 8 mm | 2,2 mm | 4,5 mm | 2,4 mm | 3,7 mm | 3,6 mm | 3,0 mm |
| d(x₅ - SZGv) = 10 mm | 1.8 mm | 2,8 mm | 2,0 mm | 2,4 mm | 2,9 mm | 2,3 mm |
| d(Wurzelspitze - SZGv) | 11,0 mm | | 11,2 mm | | 12,0 mm | |
| Wurzelmerkmal | --- | | Die letzten 4 mm 7° nach mesial geneigt | | --- | |

**Tabelle 8:**

| | | |
|---|---|---|
| Zahn | 31 | |
| Breite (Sollkontaktpkte.) | 5,5 mm | |
| Zahnlänge | 22,2 mm | |
| d(Zahnspitze - SZGv) | 8,5 mm | |

| | mesio-distal | vestibulo-oral |
|---|---|---|
| d(x₀ - SZGv) = 0 mm | 3,7 mm | 5,7 mm |
| d(x₁ - SZGv) = 2 mm | 3,5 mm | 5,8 mm |
| d(x₂ - SZGv) = 4 mm | 3,3 mm | 5,7 mm |
| d(x₃ - SZGv) = 6 mm | 3,1 mm | 5,4 mm |
| d(x₄ - SZGv) = 8 mm | 2,7 mm | 4,8 mm |
| d(x₅ - SZGv) = 10 mm | 2,3 mm | 4,2 mm |
| d(x₆ - SZGv) = 12 mm | 1,4 mm | 2,5 mm |
| d(Wurzelspitze - SZGv) | 13,7 mm | |
| Wurzelmerkmal | --- | |

**Tabelle 9:**

| | | |
|---|---|---|
| Zahn | 32 | |
| Breite (Sollkontaktpkte.) | 6,2 mm | |
| Zahnlänge | 22,3 mm | |
| d(Zahnspitze - SZGv) | 8,8 mm | |

| | mesio-distal | vestibulo-oral |
|---|---|---|
| d(x₀ - SZGv) = 0 mm | 3,7 mm | 6,4 mm |
| d(x₁ - SZGv) = 2 mm | 3,4 mm | 6,5 mm |
| d(x₂ - SZGv) = 4 mm | 3,0 mm | 6,5 mm |
| d(x₃ - SZGv) = 6 mm | 2,6 mm | 6,0 mm |
| d(x₄ - SZGv) = 8 mm | 2,3 mm | 5,0 mm |
| d(X₅ - SZGv) = 10 mm | 1,8 mm | 3,8 mm |
| d(x₆ - SZGv) = 12 mm | 1,4 mm | 2,0 mm |
| d(Wurzelspitze - SZGv) | 13,5 mm | |
| Wurzelmerkmal | Die letzten 2 mm 7° nach distal geneigt | |

**Tabelle 10:**

| | | |
|---|---|---|
| Zahn | 33 | |
| Breite (Sollkontaktpkte.) | 6,8 mm | |
| Zahnlänge | 24,3 mm | |
| d(Zahnspitze - SZGv) | 10,1 mm | |

| | mesio-distal | vestibulo-oral |
|---|---|---|
| d(x₀ - SZGv) = 0 mm | 4,5 mm | 7,1 mm |
| d(x₁ - SZGv) = 2 mm | 4,2 mm | 7,2 mm |
| d(x₂ - SZGv) = 4 mm | 3,8 mm | 7,0 mm |
| d(x₃ - SZGv) = 6 mm | 3,5 mm | 6,2 mm |
| d(x₄ - SZGv) = 8 mm | 3,2 mm | 5,5 mm |
| d(x₅ - SZGv) = 10 mm | 2,7 mm | 4,6 mm |
| d(x₆ - SZGv) = 12 mm | 2,3 mm | 3,0 mm |
| d(Wurzelspitze - SZGv) | 14,2 mm | |
| Wurzelmerkmal | Die letzten 8 mm 2° nach distal geneigt | |

**Tabelle 11:**

| | | |
|---|---|---|
| Zahn | 34 | |
| Breite (Sollkontaktpkte.) | 7,8 mm | |
| Zahnlänge | 23,2 mm | |
| d(Zahnspitze - SZGv) | 8,2 mm | |

| | mesio-distal | vestibulo-oral |
|---|---|---|
| d(x₀ - SZGv) = 0 mm | 4,8 mm | 7,0 mm |
| d(x₁ - SZGv) = 2 mm | 4,5 mm | 7,0 mm |
| d(x₂ - SZGv) = 4 mm | 4,1 mm | 6,7 mm |
| d(x₃ - SZGv) = 6 mm | 3,5 mm | 6,4 mm |
| d(x₄ - SZGv) = 8 mm | 3,3 mm | 5,4 mm |
| d(x₅ - SZGv) = 10 mm | 3,0 mm | 4,4 mm |
| d(x₆ - SZGv) = 12 mm | 2,1 mm | 3,3 mm |
| d(x₇ - SZGv) = 14 mm | 1,2 mm | 2,4 mm |
| d(Wurzelspitze - SZGv) | 15,0 mm | |
| Wurzelmerkmal | Die letzten 4 mm 2° nach distal geneigt | |

**Tabelle 12:**

| | | |
|---|---|---|
| Zahn | 35 | |
| Breite (Sollkontaktpkte.) | 7,7 mm | |
| Zahnlänge | 23,2 mm | |
| d(Zahnspitze - SZGv) | 10,2 mm | |

| | mesio-distal | vestibulo-oral |
|---|---|---|
| d(x₀ - SZGv) = 0 mm | 5,3 mm | 7,4 mm |
| d(x₁ - SZGv) = 2 mm | 4,7 mm | 7,1 mm |
| d(x₂ - SZGv) = 4 mm | 4,1 mm | 6,5 mm |
| d(x₃ - SZGv) = 6 mm | 3,6 mm | 6,0 mm |
| d(x₄ - SZGv) = 8 mm | 3,3 mm | 5,4 mm |
| d(x₅ - SZGv) = 10 mm | 2,5 mm | 4,2 mm |
| d(x₆ - SZGv) = 12 mm | 1,7 mm | 3,1 mm |
| d(Wurzelspitze - SZGv) | 13,0 mm | |
| Wurzelmerkmal | Die letzten 4 mm 30° nach distal geneigt | |

**Tabelle 13:**

| | | | | |
|---|---|---|---|---|
| Zahn | 36 | | | |
| Breite (Sollkontaktpkte.) | 11,6 mm | | | |
| Zahnlänge | 19,3 mm | | | |
| d(Zahnspitze - SZGv) | 6,4 mm | | | |

| | mesio-distal | | vestibulo-oral | |
|---|---|---|---|---|
| d(x₀ - SZGv) = 0 mm | 9,9 mm | | 9,1 mm | |
| d(x₁ - SZGv) = 2 mm | 9,3 mm | | 8,4 mm | |
| d(x₂ - SZGv) = 4 mm | 9,2 mm | | 7,9 mm | |
| d(SZGv - Furkation) | 4,2 mm | | | |
| Anzahl der Wurzeln | 2 | | | |

| | Wurzel 1 | | Wurzel 2 | |
|---|---|---|---|---|
| | mesio-distal | vestibulo-oral | mesio-distal | vestibulo-oral |
| Wurzelachse (Δx,Δy) | 0,0 mm | 0,0 mm | 3,8 mm | 0,0 mm |
| d(x₃ - SZGv) = 6 mm | 3,4 mm | 7,2 mm | 3,5 mm | 6,3 mm |
| d(x₄ - SZGv) = 8 mm | 2,8 mm | 6,4 mm | 3,0 mm | 5,0mm |
| d(x₅ - SZGv) = 10 mm | 2,3 mm | 4,6 mm | 2,6 mm | 3,5 mm |
| d(x₆ - SZGv) = 12 mm | 1,2 mm | 2,4 mm | | |
| d(Wurzelspitze - SZGv) | 12,9 mm | | 11,8 mm | |
| Wurzelmerkmal | Die letzten 4 mm 18° nach oral geneigt | | --- | |

**Tabelle 14:**

| | | | | |
|---|---|---|---|---|
| Zahn | 37 | | | |
| Breite (Sollkontaktpkte) | 10,7 mm | | | |
| Zahnlänge | 17,8 mm | | | |
| d(Zahnspitze - SZGv) | 6,0 mm | | | |

| | mesio-distal | | vestibulo-oral | |
|---|---|---|---|---|
| d(x₀ - SZGv) = 0 mm | 9,0 mm | | 8,5 mm | |
| d(x₁ - SZGv) = 2 mm | 8,8 mm | | 7,8 mm | |
| d(SZGv - Furkation) | 3,5 mm | | | |
| Anzahl der Wurzeln | 2 | | | |

| | Wurzel 1 | | Wurzel 2 | |
|---|---|---|---|---|
| | mesio-distal | vestibulo-oral | mesio-distal | vestibulo-oral |
| Wurzelachse (Δx,Δy) | 0,0 mm | -0,5 mm | 3,5 mm | -1,0 mm |
| d(x₂ - SZGv) = 4 mm | 3,4 mm | 7,3 mm | 4,4 mm | 5,5 mm |
| d(x₃ - SZGv) = 6 mm | 2,8 mm | 6,7 mm | 3,5 mm | 4,7 mm |
| d(x₄ - SZGv) = 8 mm | 2,4 mm | 5,3 mm | 3,1 mm | 3,8 mm |
| d(x₅ - SZGv) = 10 mm | 2,1 mm | 3,7 mm | 2,3 mm | 3,0 mm |
| d(Wurzelspitze - SZGv) | 11,8 mm | | 10,5 mm | |
| Wurzelmerkmal | Die letzten 4 mm 18° nach oral geneigt | | --- | |

**Tabelle 15:**

| | | |
|---|---|---|
| Zahn | 38 | |
| Breite (Sollkontaktpkte.) | 10,0 mm | |
| Zahnlänge | 15,5 mm | |
| d(Zahnspitze - SZGv) | 6,0 mm | |

| | mesio-distal | vestibulo-oral |
|---|---|---|
| d(x₀ - SZGv) = 0 mm | 8,1 mm | 8,0 mm |
| d(x₁ - SZGv) = 2 mm | 7,3 mm | 7,3 mm |
| d(x₂ - SZGv) = 4 mm | 6,0 mm | 6,3 mm |
| d(x₃ - SZGv) = 6 mm | 4,9 mm | 4,8 mm |
| d(x₄ - SZGv) = 8 mm | 3,1 mm | 3,9 mm |
| d(Wurzelspitze - SZGv) | 9,5 mm | |
| Wurzelmerkmal | --- | |

## Patentansprüche

1. Computer-implementiertes Verfahren zum Erzeugen eines ergänzten 3D-Datenmodells (10"') eines Kiefers (12, 14) eines Patienten,
wobei das ergänzte 3D-Datenmodell (10"') des Kiefers (12, 14) wenigstens ein 3D-Teildatenmodell (16) der Zahnkronen (K) der sich in einer vorbestimmten Zahnstellung befindenden Zähne (Z) des Kiefers (K) und ein 3D-Teildatenmodell (26) eines fiktiven Zahnfleischs (F_{mod}) umfasst,
wobei Ausgangspunkt des Verfahrens ein durch einen 3D-Scan erhaltenes 3D-Datenmodell (10) des Kiefers (12, 14) ist, das 3D-Teildatenmodelle (16) der Zahnkronen (K) sowie ein 3D-Teildatenmodell (18) eines tatsächlichen Zahnfleischs (Fᵢₛₜ) umfasst,
wobei ausgehend von diesem 3D-Datenmodell (10) unter Berücksichtigung einer Zahn-Zahnfleisch-Grenze (ZFG) das 3D-Teildatenmodell (18) des tatsächlichen Zahnfleischs (Fᵢₛₜ) von den 3D-Teildatenmodellen (16) der Zahnkronen (K) getrennt und schließlich entfernt wird, sodass es im Verlauf der Durchführung des Verfahrens einen Zeitpunkt gibt, zu welchem ein 3D-Datenmodell (10") des Kiefers (12, 14) existiert, das frei von jedwedem, das tatsächliche Zahnfleisch (Fᵢₛₜ) des Patienten repräsentierendem 3D-Teildatenmodell (18) ist und das eine vorbestimmte Zahnstellung der Zähne (Z) beinhaltet, und
wobei anschließend ein 3D-Teildatenmodell (26) eines fiktiven Zahnfleischs (F_{mod}) auf Grundlage zumindest des 3D-Teildatenmodells (16) der Zahnkronen (K) erstellt und zu dem 3D-Datenmodell (10"), das die vorbestimmte Zahnstellung der Zähne (Z) beinhaltet, hinzugefügt wird,
**dadurch gekennzeichnet, dass**
beim Erstellen des 3D-Datenmodells (26) des fiktiven Zahnfleischs (F_{mod}) für jeden der Zähne (Z) des 3D-Datenmodells (10"), das die vorbestimmte Zahnstellung der Zähne (Z) beinhaltet, gesondert ein 3D-Datenmodell (28) einer Basisgingiva (BG) erstellt wird und
die 3D-Datenmodelle (28) der Basisgingivae (BG) aller Zähne (Z) mit dem 3D-Datenmodell (10"), das die vorbestimmte Zahnstellung der Zähne (Z) beinhaltet, zu dem ergänzten 3D-Datenmodell (10'") des Kiefers (12, 14) verbunden werden.

2. Verfahren nach Anspruch 1, **dadurch gekennzeichnet, dass** beim Erstellen des 3D-Datenmodells (28) der Basisgingiva (BG) jedes Zahns (Z) zumindest von wenigstens zwei charakteristischen Abmessungen einer freien marginalen Gingiva (F_{FMG}) jedes Zahns (Z) und wenigstens einer charakteristischen Abmessung einer eine Zahnwurzel (W) umgebenden Gingiva (F_{w}) ausgegangen wird.

3. Verfahren nach Anspruch 2, **dadurch gekennzeichnet, dass** das 3D-Datenmodell (28) der Basisgingiva (BG) jedes Zahns (Z) unter Verwendung von durch die charakteristischen Abmessungen vorgegebenen 3D-Datenpunkten als Stützpunkte mittels eines interpolierenden und/oder aproximierenden Verfahrens erzeugt werden, beispielsweise mittels eines Verfahrens der impliziten Oberflächen (implicit surfaces).

4. Verfahren nach einem der Ansprüche 1 bis 3, **dadurch gekennzeichnet, dass** nach dem Erstellen des 3D-Datenmodells (28) der Basisgingiva (BG) eines Zahns (Z) im Inneren dieses 3D-Datenmodells (28) angeordnete 3D-Datenpunkte aus diesem eliminiert werden.

5. Verfahren nach einem der Ansprüche 1 bis 4, **dadurch gekennzeichnet, dass** beim Verbinden der 3D-Datenmodelle (28) der Basisgingivae (BG) aller Zähne (Z) miteinander und mit dem 3D- Datenmodell (10ⁱⁱ) der vorbestimmten Zahnstellung zu dem ergänzten 3D- Datenmodell (10ⁱⁱⁱ) der vorbestimmten Zahnstellung im Inneren des ergänzten 3D-Datenmodells angeordnete 3D-Datenpunkte aus diesem eliminiert werden.

6. Verfahren nach einem der Ansprüche 1 bis 5, **dadurch gekennzeichnet, dass** die 3D-Datenmodelle (28) der Basisgingiva (BG) einander benachbarter Zähne (Z) mittels 3D-Datenmodellen (30) konkaver Abflachungsabschnitte (32) verbunden werden.

7. Verfahren nach Anspruch 6, **dadurch gekennzeichnet, dass** ein zwischen zwei benachbarten Zähnen (Z) vorgesehener, vestibulärer oder lingualer Abflachungsabschnitt (32) auf jeder Höhe von einer auf dieser Höhe verlaufenden und die Basisgingiva (BG) der beiden benachbarten Zähne (Z) tangential berührenden Verbindungslinie (VL) einen vorbestimmten Maximalabstand (dₘₐₓ) aufweist.

8. Verfahren nach Anspruch 6 oder 7, **dadurch gekennzeichnet, dass** ein zwischen zwei benachbarten Zähnen (Z) vorgesehener, okklusaler Abflachungsabschnitt von einer auf oberen Rändern der freien marginalen Gingiva (F_{FMG}) der beiden benachbarten Zähne aufliegenden Verbindungslinie einen vorbestimmten Maximalabstand aufweist.

9. Verfahren nach einem der Ansprüche 1 bis 8, **dadurch gekennzeichnet, dass** die Gingiva distal zweier endständiger Zähne (Z_{end}) des jeweiligen Kiefers (12) bzw. (14) um eine vorbestimmte Länge (l_{end}) fortgesetzt wird, vorzugsweise in einem vorbestimmten Abstand (h_{end}) von der Okklusalebene (OE) und mit einer vorbestimmten Dicke (b_{end}).

10. Verfahren nach einem der Ansprüche 1 bis 9, **dadurch gekennzeichnet, dass** man die Gingiva (F_{mod}) nur bis zu einer Umschlagfalte (UF) modelliert.

11. Verfahren nach einem der Ansprüche 1 bis 10, **dadurch gekennzeichnet, dass** man für den Oberkiefer (12) ein 3D-Datenmodell (38) eines sich zwischen zwei Zahnbogenhälften erstreckenden Gaumens (G) erstellt.

12. Verfahren nach Anspruch 11, **dadurch gekennzeichnet, dass** am 3D-Datenmodell (38) des Gaumens (G) wenigstens eine ergänzende Struktur (40) vorgesehen wird, welche beispielsweise die Papilla incisiva oder/und die Plicae transversae oder/und die Satura palatina oder/und das Lippenbändchen oder/und wenigstens eines der Wangenbändchen repräsentiert.

13. Verfahren nach einem der Ansprüche 1 bis 12, **dadurch gekennzeichnet, dass** am 3D-Datenmodell des Unterkiefers (14) wenigstens eine ergänzende Struktur vorgesehen wird, welche beispielsweise das Zungenbändchen oder/und das Lippenbändchen oder/und wenigstens eines der Wangenbändchen repräsentiert.

14. Verfahren nach einem der Ansprüche 1 bis 13, **dadurch gekennzeichnet, dass** nach der Erstellung des 3D-Datenmodells (26) des fiktiven Zahnfleischs (F_{mod}) an diesem Oberflächencharakteristika des tatsächlichen Zahnfleischs (Fᵢₛₜ) vorgesehen werden.

## Claims

1. A computer-implemented method for creating an expanded 3D data model (10''') of a jaw (12, 14) of a patient,
the expanded 3D data model (10''') of the jaw (12, 14) comprising at least one partial 3D data model (16) of the dental crowns (K) of the teeth (Z) of the jaw (K), which are located in a predetermined tooth position, and a partial 3D data model (26) of a fictitious gum (F_{mod)},
the starting point of the method being a 3D data model (10) of the jaw (12, 14) obtained by means of a 3D scan, which comprises partial 3D data models (16) of the dental crowns (K) and a partial 3D data model (18) of an actual gum (F_{act}),
starting from this 3D data model (10) and taking account of a tooth/gum boundary (ZFG), the partial 3D data model (18) of the actual gum (F_{act})being separated and finally removed from the partial 3D data models (16) of the dental crowns (K), so that in the course of carrying out the method, there is a time at which a 3D data model (10") of the jaw (12, 14) exists, which is free from any partial 3D data model (18) representing the actual gum (F_{act}) of the patient and which contains a predetermined tooth position of the teeth (Z), and
subsequently, a partial 3D data model (26) of a fictitious gum (F_{mod}) being created on the basis of at least the partial 3D data model (16) of the dental crowns (K) and added to the 3D data model (10''), which contains the predetermined tooth position of the teeth (Z),
**characterized in that**
when creating the 3D data model (26) of the fictitious gum (F_{mod}) for each of the teeth (Z) of the 3D data model (10"), which contains the predetermined tooth position of the teeth (Z), a 3D data model (28) of a basic gingiva (BG) is created separately, and
the 3D data models (28) of the basic gingivae (BG) of all teeth (Z) are connected to the 3D data model (10''), which contains the predetermined tooth position of the teeth (Z), to give the expanded 3D data model (10''') of the jaw (12, 14).

2. The method according to Claim 1, **characterized in that**, when creating the 3D data model (28) of the basic gingiva (BG) of each tooth (Z), at least two
characteristic dimensions of a free marginal gingiva (F_{FMG}) of each tooth (Z) and at least one characteristic dimension of a gingiva (F_{W}) surrounding a tooth root (W), at least, are taken as a starting point.

3. The method according to Claim 2, **characterized in that** the 3D data model (28) of the basic gingiva (BG) of each tooth (Z) is created using 3D data points predetermined by the characteristic dimensions as support points by means of an interpolating and/or approximating method, for example by means of an implicit surface method.

4. The method according to one of Claims 1 to 3,
**characterized in that**, after the creation of the 3D data model (28) of the basic gingiva (BG) of a tooth (Z), 3D data points arranged in the interior of this 3D data model (28) are eliminated from the same.

5. The method according to one of Claims 1 to 4,
**characterized in that**, when connecting the 3D data models (28) of the basic gingivae (BG) of all teeth (Z) to one another and to the 3D data model (10ⁱⁱ) of the predetermined tooth position to give the expanded 3D data model (10ⁱⁱⁱ) of the predetermined tooth position, 3D data points arranged in the interior of the expanded 3D data model are eliminated from the same.

6. The method according to one of Claims 1 to 5,
**characterized in that** the 3D data models (28) of the basic gingiva (BG) of mutually adjacent teeth (Z) are connected by means of 3D data models (30) of concave flattening sections (32).

7. The method according to Claim 6, **characterized in that** a vestibular or lingual flattening section (32) provided between two adjacent teeth (Z) has a predetermined maximum distance (dₘₐₓ) at any height from a connecting line (VL) running at this height and tangentially touching the basic gingiva (BG) of the two adjacent teeth (Z).

8. The method according to Claim 6 or 7, **characterized in that** an occlusal flattening section provided between two adjacent teeth (Z) has a predetermined maximum distance from a connecting line resting on upper edges of the free marginal gingiva (F_{FMG}) of the two adjacent teeth.

9. The method according to one of Claims 1 to 8,
**characterized in that** the gingiva is continued distally to two terminal teeth (Z_{end}) of the respective jaw (12) or (14) by a predetermined length (l_{end}), preferably at a predetermined distance (h_{end}) from the occlusal plane (OE) and with a predetermined thickness (b_{end}).

10. The method according to one of Claims 1 to 9,
**characterized in that** one only models the gingiva (F_{mod}) up to a vestibule (UF).

11. The method according to one of Claims 1 to 10,
**characterized in that**, for the upper jaw (12), one creates a 3D data model (38) of a palate (G) extending between two dental arch halves.

12. The method according to Claim 11, **characterized in that** at least one expanding structure (40) is provided on the 3D data model (38) of the palate (G), which structure for example represents the papilla incisiva and/or the plicae transversae and/or the palatine suture and/or the frenulum of the lip and/or at least one of the frenula of the cheeks.

13. The method according to one of Claims 1 to 12,
**characterized in that** on the 3D data model of the lower jaw (14), at least one expanding structure is provided, which for example represents the frenulum of the tongue and/or the frenulum of the lip and/or at least one of the frenula of the cheeks.

14. The method according to one of Claims 1 to 13,
**characterized in that** following the creation of the 3D data model (26) of the fictitious gum (F_{mod}), surface characteristics of the actual gum (F_{act}) are provided on the same.

## Revendications

1. Procédé implémenté par ordinateur pour produire un modèle 3D complété (10"') d'une mâchoire (12, 14) d'un patient,
dans lequel le modèle 3D complété (10''') de la mâchoire (12, 14) comprend au moins un modèle de données partiel 3D (16) des couronnes dentaires (K) des dents (Z) de la mâchoire (K) se trouvant dans un alignement dentaire prédéterminé et un modèle de données partiel 3D (26) d'une gencive fictive (F_{mod}),
dans lequel le point de départ du procédé est un modèle de données 3D (10) de la mâchoire (12, 14) obtenu par un scan 3D, le modèle de données partiel 3D (16) des couronnes dentaires (K) ainsi qu'un modèle de données partiel 3D (18) d'une gencive effective (Fᵢₛₜ),
dans lequel à partir de ce modèle de données 3D (10) en prenant en compte une limite dent-gencive (ZFG) le modèle de données partiel 3D (18) de la gencive effective (Fᵢₛₜ) est séparé des modèles de données partiels (16) des couronnes dentaires (K) et finalement éliminé, dès qu'un point temporel apparait au cours de la mise en oeuvre du procédé, auquel un modèle de données 3D (10'') de la mâchoire (12, 14) existe, qui est exempt d'un quelconque modèle de données partiel 3D (18) représentant la gencive effective (Fᵢₛₜ) du patient et qui contient un alignement dentaire des dents (Z) prédéterminé, et
dans lequel ensuite un modèle de données partiel 3D (26) d'une gencive fictive (F_{mod}) est établi en se basant sur au moins le modèle de données partiel 3D (16) des couronnes dentaires (K) et auquel est ajouté le modèle de données 3D (10''), qui contient l'alignement dentaire prédéterminé des dents (Z),
**caractérisé en ce que**
lors de l'établissement du modèle de données 3D (26) de la gencive fictive (F_{mod}) pour chacune des dents (Z) du modèle de données 3D (10''), qui contient l'alignement dentaire prédéterminé des dents (Z), un modèle 3D (28) d'une gencive de base (BG) est spécialement établi et
les modèles de données 3D (28) de la gencive de base (BG) de toutes les dents (Z) sont reliés au modèle de données 3D (10"), qui contient l'alignement dentaire prédéterminé des dents (Z), afin d'obtenir le modèle de données 3D complété (10''') de la mâchoire (12, 14).

2. Procédé selon la revendication 1, **caractérisé en ce que** lors de l'établissement du modèle de données 3D (28) de la gencive de base (BG) de chaque dent (Z), on se base au départ sur au moins deux dimensions caractéristiques d'une gencive marginale libre (F_{FMG}) de chaque dent (Z) et au moins une dimension
caractéristique d'une gencive (F_{w}) entourant une racine de dent (W).

3. Procédé selon la revendication 2, **caractérisé en ce que** le modèle de données 3D (28) de la gencive de base (BG) de chaque dent (Z) est produit en utilisant les points de données 3D prescrits par les dimensions
caractéristiques comme points d'appui au moyen d'une procédé d'interpolation et/ou d'approximation, par exemple au moyen d'un procédé de surfaces implicites.

4. Procédé selon une des revendications 1 à 3,
**caractérisé en ce que** après l'établissement du modèle de données 3D (28) de la gencive de base (BG) d'une dent (Z) des points de donnée s3D disposés à l'intérieur de ce modèle de données 3D (28) sont éliminés de ce dernier.

5. Procédé selon une des revendications 1 à 4,
**caractérisé en ce que** lors de la liaison des modèles de données 3D (28) des gencives de base (BG) de toutes les dents (Z) les uns avec les autres et avec le modèle de données 3D (10ⁱⁱ) de l'alignement dentaire prédéterminé afin de former le modèle de données 3D complété (10ⁱⁱⁱ) de l'alignement dentaire prédéterminé, des points de données 3D disposés à l'intérieur du modèle de données 3D complété sont éliminés de celui-ci.

6. Procédé selon une des revendications 1 à 5,
**caractérisé en ce que** les modèles de données 3D (28) des gencives de base (BG) des dents (Z) voisines les unes des autres sont reliées au moyen de modèles de données 3D (30) de portions de méplat concaves (32).

7. Procédé selon la revendication 6, **caractérisé en ce que** une portion de méplat (32) vestibulaire ou linguale ligne de liaison (VL) tangentielle prévue entre deux dents voisines (Z), sur chaque hauteur d'une ligne de liaison (VL) s'étendant sur une de ces hauteurs et contactant tangentiellement la gencive de base (BG) des deux dents voisines (Z) présent un écart maximal (dₘₐₓ) prescrit.

8. Procédé selon la revendication 6 ou 7, **caractérisé en ce que** une surface de méplat occlusale, prévue entre deux dents voisines (Z) d'une ligne de liaison située sur les bords supérieures de la gencive marginale libre (F_{FMG}) des deux dents voisines présente un écart maximal prédéterminé.

9. Procédé selon une des revendications 1 à 8,
**caractérisé en ce que** la gencive des dents en fin de dentition (Z_{end}) de la mâchoire respective (12), respectivement (14) est avancée distalement d'une longueur prescrite (I_{end}), de préférence dans un écart prescrit (h_{end}) par rapport au plan occlusal (OE) et avec une épaisseur prescrite (b_{end}).

10. Procédé selon une des revendications 1 à 9,
**caractérisé en ce que** on modélise la gencive (F_{mod}) seulement jusqu'à un repli vestibulaire (UF).

11. Procédé selon une des revendications 1 à 10,
**caractérisé en ce que** on établit pour le maxillaire (12) un modèle de données 3D (38) d'un palais (G) s'étendant entre deux moitiés d'arcade dentaire.

12. Procédé selon la revendication 11, **caractérisé en ce que** sur le modèle de données 3D (38) du palais (G) au moins une structure complémentaire (40) est prévue, qui représente par exemple la papille incisive ou/et les plis transverses ou/et la suture palatine ou/et le frein labial ou/et au moins un des freins vestibulaires.

13. Procédé selon une des revendications 1 à 12,
**caractérisé en ce que** sur le modèle de données 3D de la mandibule (14) au moins une structure complémentaire est prévue, qui représente par exemple le frein lingual ou/et le frein labial ou/et au moins un des freins mandibulaires.

14. Procédé selon une des revendications 1 à 13,
**caractérisé en ce que** après l'établissement du modèle de données 3D (26) de la gencive fictive (F_{mod}) sur ce dernier des caractéristiques de surface de la gencive effective (Fᵢₛₜ) sont prévues.
